# EUROPEAN PATENT SPECIFICATION

(11) **EP 3 154 495 B1**
(45) Date of publication and mention of the grant of the patent: **21.11.2018**
(21) Application number: 14894692.4
(22) Date of filing: 13.06.2014
(51) Int. Cl.: A61F 13/551, B32B 38/18, B65D 65/14, B65D 75/20, B65H 23/00

(54) **A PACKAGING UNIT FOR HYGIENE ARTICLES AND A METHOD OF FORMING A PACKAGING UNIT**
VERPACKUNGSEINHEIT FÜR HYGIENEARTIKEL UND VERFAHREN ZUR HERSTELLUNG EINER VERPACKUNGSEINHEIT
UNITÉ DE CONDITIONNEMENT POUR ARTICLES D'HYGIÈNE ET PROCÉDÉ DE FORMATION D'UNE UNITÉ DE CONDITIONNEMENT

(43) Date of publication of application: 19.04.2017
(73) Proprietor: Essity Hygiene and Health Aktiebolag, 405 03 Göteborg (SE)
(72) Inventor: HOLMBERG, Anders, S-405 03 Göteborg (SE)
(74) Representative: Valea AB
(86) International application number: PCT/SE2014/050724
(87) International publication number: WO 2015/190968

(56) References cited:
- WO-A1-2013/162430
- JP-A- 2006 045 417
- US-A- 5 591 153
- US-A1- 2003 234 069
- US-A1- 2009 082 747
- US-A1- 2010 175 825
- US-A1- 2012 090 071
- US-A1- 2012 283 682
- US-A1- 2013 199 956

## Description

### TECHNICAL FIELD

The present invention relates to a packaging unit for hygiene articles. The present invention also relates to a blank of an elongated sheet material for providing a packaging unit, as well as to a method of forming a packaging unit for hygiene articles from a blank of an elongated sheet material.

### BACKGROUND

Disposable hygiene articles, such as sanitary napkins and panty liners, are normally packaged individually in, for example, an easy wrap or a single wrap. Individual packages facilitate hygienic carrying of single articles for future use, e.g. in a handbag. The edges of the individual packages can be sealed by means such as ultrasonic welding, heat welding and mechanical embossing with or without heat. Further, the packaging units are often used both as a means for packaging an unused article and for disposal of the used article.

It is desirable that used articles of this kind can be disposed of discretely and hygienically. This may be particularly important when the user lacks the possibility to dispose of the used article immediately after the used article has been replaced, e.g. when there is no waste bin available in the toilet area. In this case, the user may need to put the used article in e.g. a handbag or backpack, which requires that the package is adequately sealed in order to avoid staining and odour.

To solve the problem of providing a single wrap that may be both used for packaging of a new and unused hygiene article and for safe and hygienic disposal of used hygiene articles, the use of "post-it-like" adhesives has been shown to be useful as sealing means for the individual wrap packages.

WO2013/162430 A1 discloses such a packaging unit in the form a single wrap that can be used both for packaging of a new and unused hygiene article and for safe and hygienic disposing of used hygiene articles. The packaging unit is in the form of a sheet provided with adhesive in a certain chessboard pattern for closing and sealing the packaging unit. Such a sheet may be fabricated by providing a sheet material on which, for example, adhesive is applied, and cutting the sheet into individual sheet of materials for forming the packaging unit.

Although said prior art packaging unit to some extent may alleviate the problems of providing a safe and hygienic packaging for unused as well as used hygiene articles, there is still a problem of providing adequate closure and sealing of the package by the use of adhesive, wherein it may be difficult to adhere the adhesive to a sheet surface during production of the packaging unit as well as during the use of the packaging unit.

Thus, there is still a need for further improvements of packaging units so as to provide a packaging unit that is easy and reliable to produce and handle, wherein the packaging unit may adequately enclose and seal an unused and a used hygiene article.

### SUMMARY

In view of known packaging units, it is an object of the present invention to provide an improved packaging unit arrangement for carrying new hygiene articles as well as used hygiene articles, which arrangement provides easy and reliable handling, while being easy to produce.

The object is wholly or partially achieved by a packaging unit according to appended claim 1, a blank according to claim 21 and a method according to appended claim 23. Embodiments are set forth in the appended dependent claims, in the following description and in the drawings.

Thus, there is provided a packaging unit for hygiene articles, the unit being formed from a sheet of material having a longitudinal direction (L) and a transverse direction (T). The sheet has an inner surface and an outer surface, wherein the inner surface comprises an edge zone that comprises an edge portion being provided with a resealable adhesive for closure of the packaging unit. The sheet has at least one transversely extending folding axis dividing the sheet into a first region and a second region. Furthermore, the sheet is provided with a sync mark for indication of a positioning of the sheet of material during manufacturing of the packaging unit. The sync mark is provided on the outer surface. The packaging unit is in the form of a sheet that may be used as a single wrap that has resealable adhesive for closure of the packaging unit holding an unused a hygiene article therein or a used article therein.

As used herein, the term "inner surface" refers to the surface of the packaging unit facing the product positioned inside the packaging unit, and the term "outer surface" refers to the surface opposite to the inner surface, i.e. the surface facing the ambient.

By the term "edge zone" is meant the portion of the packaging unit adjacent to the edges of the packaging unit. The width of an edge zone may be varied. The edge zone may be divided into an inner edge portion and an outer edge portion. The term "inner edge portion" then refers to the portion of the edge zone positioned towards center of the packaging unit, such as towards the longitudinal center line of the packaging unit. The term "outer edge portion" refers to the portion of the edge zone positioned towards the edge of the packaging unit.

By a "resealable adhesive" as used herein is meant adhesive that provides that an object may be attached to a surface using such an adhesive and later on detached from that surface causing minor or no disruptions of the object and the surface. Furthermore, an object that has been peeled off may also be reattached to a surface again.

By the term "sync mark" is meant a position mark for synchronizing a positioning of the packaging unit and its parts during manufacturing thereof.

The sync mark provided on the outer surface may therefore be used as a mark for discerning the individual packing unit and regions thereof during production thereof. For example, it may be used as a positioning mark for indicating a cutting position of a blank sheet of material for providing a single packaging unit such as a wrap. Also, the sync mark may be used as a mark for examining whether, for example, the resealable adhesive is applied at a predetermined position on the packaging unit.

By providing the sync mark on the outer surface, an improved resealable adhesive connection to the inner surface is made possible and with no sync marks on the inner surface, the inner surface is also prepared for pre-treatments such as corona treatments for providing an improved resealable adhesive connection to the inner surface.

The sync mark may be a printed mark.

A printed mark is a mark printed to form a coloured mark, including a mark in black. The printed mark may be provided by a contact printing technique such as flexographic printing, rotogravure printing, screen printing and offset printing. The printed mark may also be provided by a non-contact printing technique such as ink jet printing, wax jet printing, bubble jet printing and laser jet printing.

At least a portion of the inner surface may have been pre-treated with a surface treatment for changing the surface energy.

The surface energy level corresponds to properties of the surface, in particular to the disruption of intermolecular bonds that occur when a surface is created or pre-treated. The pre-treatment modifies the surface properties, such as increasing the disruption of intermolecular bonds, and thereby increases the surface energy that in turn provides an improved adhesive connection.

The pre-treated portion may overlap with the edge portion comprising the resealable adhesive for closure of the packaging unit.

The surface treatment may be a plasma treatment. The plasma treatment may be a corona treatment. The corona treatment may be performed as is known in the field and described in US 2012/0232509 A1 wherein the treatment is applied at from 1 to 10, from 3 to 5 or 4 watts per square feet per minute. The corona-treated sheet of material should be subjected to application of adhesives and other agents shortly after the corona treatment of the sheet, preferably within 2 to 6 weeks from the corona treatment has been performed.

"Corona treatment" is a surface treatment technique that uses the so-called corona discharge plasma by the application of high voltage at low temperature to modify the properties of a surface. Materials such as plastics and nonwoven substrates may be corona treated so as to change the surface energy of such materials and to provide better bonding surfaces. The corona treatment increases the surface energy of the material and thereby improving the wettability and adhesion characteristics of, for example, adhesives to the materials such as polyolefin films and nonwoven substrates.

By providing the sync mark on the outer surface and pre-treating the inner surface with a corona treatment, there is provided an adequate adhesive connection to the corona-treated surface, as compared to providing the sync mark on the inner surface since any printing on the inner surface tends to interfere with the adhesive attachment, as the attachment to a corona-treated surface is stronger than to a printed surface, even if the printed surface portion has been corona treated.

A portion of the resealable adhesive for closure of the packaging unit may be in contact with a portion of the surface that has been pre-treated with surface treatment.

The complete inner surface portion to which the resealable adhesive is applied may be corona treated. The complete inner surface of the packaging unit sheet may also be pre-treated.

The sheet may be folded along the at least one folding axis with the first and second regions in an overlapping configuration, thereby providing a packaging unit of overlapping sheet layers.

The sheet of material may be of substantially rectangular shape and comprise longitudinal edges and transverse edges.

The sync mark may be provided at a longitudinal edge portion and/or at a transversal edge portion of the sheet of material.

The transverse direction (T) normally corresponds to a machine direction (MD) during manufacturing of the packaging unit. The longitudinal direction (L) then corresponds to the cross machine direction (CD). The cutting of the blank sheet of material may then be performed along the longitudinal direction, i.e. CD.

As mentioned above, the packaging unit comprises at least one first folding axis. The number of folding axes may vary depending on how the packaging unit is intended to be folded. It is preferred that the packaging unit comprises between one and three folding axes. The sheet may have two folding axes that divide the sheet into the first region, the second region and a third region.

The sheet may then be folded along the folding axes with the first, second and third regions in an overlapping configuration of three layers. The first region may form an outer layer of the folded packaging unit and the third region an inner layer. The sync mark may be provided on the first region, second region or third region.

The packaging unit may comprise two or more sync marks.

The sheet may a laminate material. By the term "laminate" is meant a material comprising at least two united separable plies of material that can be the same or different. In the context of the present invention, the laminate may for example be constituted of two separable plies of plastic film, a film and nonwoven, two plies of nonwoven or the like.

The sheet may be a single-ply material.

By the term "single ply" is meant a packaging unit comprising a single ply of a coherent material. Examples of a single ply packaging unit may be a plastic film, such as a polyethylene film, a nonwoven material, a metallic foil or the like. A single ply material may be a non-homogenous material, such as a plastic film material comprising integrated layers or a nonwoven material having varying fibre composition in different parts of the material. A single ply material as used herein does not comprise materials having separable layers.

The single-ply material may be of polyolefin that may comprise or be of polyethylene and/or polypropylene. The material of polyethylene and/or polypropylene may have a basis weight of 14 to 30 g/m2.

A chessboard pattern of resealable adhesive and non-adhesive portions may be formed along each edge zones for complementary attachments between resealable adhesive and non-adhesive portions in a folded packaging unit.

Examples of different chessboard patterns will be further described below. WO2013/162430 A1 discloses further examples of such patterns.

The sheet may be opaque and/or may comprise print and/or tactile indications, other than the sync marks, preferably provided on the outer surface. Thus, the sheet may, for example, be embossed.

The print may be instructions or indications that guide the user in the manipulation of the packaging unit and/or the hygiene article.

The resealable adhesive may be a pressure-sensitive hotmelt adhesive. Such an adhesive provides unlimited open time, meaning that the adhesive can bond to another substrate at any time. It is may provide "post-it-like" adhesive properties. Thus, the packaging unit may be reclosable.

Accordingly, the pressure-sensitive adhesive used with the packaging unit is one which has a very high self-adhesion but which can be readily separated or released from other materials, such as plastic materials or paper which has been treated with a release agent. A major advantage of the packaging unit according to the present invention is that it can be completely unfolded when a new hygiene article is about to be taken out. In contrast thereto, prior art packages having adhesively sealed edges with adhesive-coated edge portions in contact with each other have too high adhesive strength of the adhesively sealed edges, and any attempt to completely unfold the package generally leads to tearing and breakage of the packaging unit, making it unusable for discrete and hygienic disposal of the used article. As the adhesive-coated edge portions of the packaging unit of the present invention are not in contact with each other when the packaging unit is folded, the packaging unit can be readily opened and resealed, providing a tight disposal package. At the same time, the tensile strength of the adhesively sealed edges of the packaging unit using the resealable adhesive pattern of the present invention is sufficient to provide a tight package for both a new and a used article, and low enough to provide a readily-opened package.

The resealable adhesive used in the present invention may be a pressure-sensitive hotmelt adhesive, such as Lunatack® D656 BD 19 available from H. B. Fuller.

The length of the adhesive-covered edge portions in each region may be equal to or shorter than the length or width of each region. The width of the adhesive-covered edge portions may be varied depending on the adhesive strength desired. The wider the adhesive-covered edge zones, the stronger the sealing. The width of the adhesive-covered edge portions may be same or different in the different regions.

The sheet of material forming a packaging unit according to the present invention may comprise an odour-inhibiting or odour-neutralising substance. Such a substance may be applied in any suitable manner known to the person skilled in the art, e.g. as a coating, activatable microcapsules, impregnated patches, or the like.

It is conceivable that the sheet for forming a packaging unit according to the present invention may be stretchable or expandable, which may be advantageous if the hygiene article is greatly deformed during use, and may thus be difficult to wrap without deforming the packaging unit.

It should be noted that when the packaging unit according to the present invention is used for disposal, the user may choose to roll up the packaging unit instead of folding, regardless of the resealable adhesive pattern at the edge zone of the packaging unit.

The inner surface may comprise a center zone that comprises a portion provided with a release agent.

The term "central zone" refers to the portion of the packaging unit excluding the edge zone and overlapping with the center of the packaging unit.

The release agent is intended for releasable connection to an adhesive of a hygiene article in the packaging unit. The release agent may be a silicone polymer or polysiloxane.

As an alternative, a release liner may be attached to the central zone of the inner surface.

Such a release liner is also intended for releasable connection to a hygiene article. The release liner may be manufactured from any suitable material known to the person skilled in the art, such as paper, non-woven or plastic film material. Further, in order to provide a release liner that is easily detachable from the garment-affixing adhesive that may be present on a garment-facing surface of the hygiene article, the release liner may be silicone-coated on the surface of the release liner that is intended to be connected to the hygiene article. The hygiene article may be releasably affixed to the release liner either by means of a resealable adhesive or by any other suitable fastening means known to the person skilled in the art, e.g. hook-and-loop fastening means.

"Releasably affixed" means two surfaces being bonded to each other such that the bond may be readily broken without affecting the surfaces.

The packaging unit may comprise a hygiene article.

The hygiene article may be arranged on the inner surface, in the center zone thereof, wherein the hygiene article may be releasably attached to the release agent via an adhesive, or to the release liner.

There is also provided a blank for manufacturing at least two packaging units as described above. The blank is an elongated sheet of material having first and second surfaces, wherein the first surface comprises the inner surface of each packaging unit and the second surface comprises the outer surface of each packaging unit. The inner surface comprises a portion with resealable adhesive for closure of the packaging unit formed from the blank and the outer surface comprises one or more sync marks.

As mentioned above, the sync mark may be used for guiding a cutting tool to cut the blank into individual sheets of material and forming the packaging units.

A release agent in the form of, for example, a dispersion or layer may be applied on the first surface and inner surface, and a hygiene article may be arranged on the release agent.

At least a portion of the inner surface may have been pre-treated with a surface treatment for increasing the surface energy before any resealable adhesive has been applied on the first surface so as to improve adhesive connection to the pre-treated portion of the first surface.

The surface treatment may be a plasma treatment, such as a corona treatment.

There is also provided a method of forming a packaging unit for hygiene articles, the unit being formed from a blank of an elongated sheet of material. The method comprises the steps of:
- providing the elongated sheet of material comprising at least two packaging unit sections, the sheet having first and second surfaces, the first surface comprising an inner surface of each packaging unit and the second surface comprising an outer surface of each packaging unit,
- providing the outer surface with one or more sync marks,
- applying a resealable adhesive on an edge zone portion of the inner surface of each packaging unit section;
- detecting the sync mark and its position on the blank, and
- cutting the elongated sheet into two or more single sheets or packaging units.

The position of cutting may be decided based on the detected position of the sync mark. Each portion with resealable adhesive may be arranged in an edge zone of a packaging unit section.

One or more sync mark detectors may be arranged to detect the sync mark on the outer surface during the manufacturing, wherein a detection of the sync mark is used for indicating a position of a blank sheet of material so as to determine, for example, a cutting position of the blank sheet of material for providing single packaging unit such as a wrap.

The method may further comprise a step of pre-treating at least a portion of the first surface with a surface treatment for increasing the surface energy.

The surface treatment may be a plasma treatment. The treatment may be a corona treatment.

The elongated sheet may comprise at least one folding axis extending in the machine direction (MD). The folding axis divides the elongated sheet into a first blank region and a second blank region. The method may further comprise folding the elongated sheet along the at least one folding axis with the first and second blank regions in an overlapping configuration, thereby providing a folded elongated sheet of overlapping sheet layers, wherein the step of cutting the elongated sheet occurs after the folding step.

The transversely extending folding axis may extend along the machine direction (MD) and the cutting may occur along the cross machine direction (CD).

Each single sheet formed after the step of cutting may comprise at least one transversely extending folding axis that divides each single sheet into a first region and a second region, wherein the method in a step following the method step of cutting the elongated sheet comprises folding the single sheet along the at least one folding axis with the first and second regions in an overlapping configuration, thereby providing a packaging unit of overlapping sheet layers.

The cutting may accordingly occur prior to the folding step. Nonetheless, the transversely extending folding axis may still extend along the machine direction (MD) and the cutting may occur along the cross machine direction (CD).

The formed packaging unit for hygiene articles may be a packaging unit as defined herein.

The method may, prior to the folding step, comprise:
- applying a release agent on the first surface on a portion of each center zone of a packaging unit section,
- applying a resealable adhesive on the portion with release agent, and
- applying a hygiene article to the adhesive applied on the release agent.

Alternatively, the method may, prior to the folding step, comprise:
- attaching a release liner on the first surface on a portion of each center zone of a packaging unit section and
- connecting a hygiene article to the release liner by the use of fastening means.

Thus, the hygiene article may be releasably affixed to a surface of the release liner after the release liner has been permanently attached to the inner surface of the packaging unit. Alternatively, the hygiene article may be releasably affixed to the user-facing surface of the release liner before the release liner has been permanently attached to the inner surface of the wrapping sheet. The hygiene article may be releasably affixed to the release liner either by means of a resealable adhesive or by any other suitable fastening means known to the person skilled in the art, e.g. hook-and-loop fastening means.

The sync mark may aid in positioning of the applied agents and articles.

The invention will now be described in more detail with reference to embodiments and drawings.

### BRIEF DESCRIPTION OF THE DRAWINGS

Fig. 1 shows a packaging unit according to the present invention having one folding axis;
Fig. 2 shows the packaging unit depicted in Fig. 1 in a folded state;
Fig. 3 shows a packaging unit according to the present invention having two folding axes;
Fig. 4 shows the packaging unit depicted in Fig. 3 in a folded state.
Fig. 5 schematically illustrates an arrangement for forming a packaging unit depicted in Figs. 3 and 4.

### DETAILED DESCRIPTION OF EMBODIMENTS

The packaging unit of the invention is in the form of a sheet that may be used as a single wrap that has resealable adhesive for closure of the packaging unit holding an unused hygiene article therein or a used article therein. The closure of the packaging unit may include folding the sheet and enclosing the hygiene article between folded sheet layers that are sealed by the resealable adhesive provided close to the edges of the sheets. Conceivable hygiene articles are sanitary napkins, panty liners and the like.

In the following, the packaging unit will be exemplified by a packaging unit comprising a rectangular sheet. Other forms such as circular sheet are also within the scope of the appended claims. Furthermore, the exemplified packaging unit comprises resealable adhesive for closure of the packaging unit, wherein a chessboard pattern of resealable adhesive and non-adhesive portions is formed along at least longitudinal edge zones (i.e. along the longitudinal direction (L)) for complementary attachment between resealable adhesive and non-adhesive portions in a folded packaging unit. According to the invention, other ways of applying one or more resealable adhesive portions for closure of the packaging unit may also be considered.

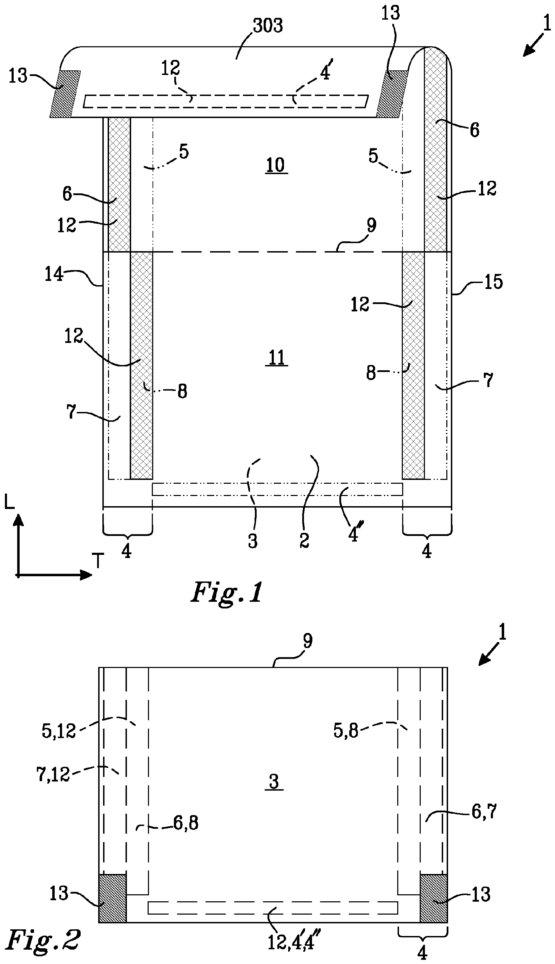

Fig. 1 depicts a packaging unit 1 for hygiene articles according to the present invention. The packaging unit is formed from a sheet of material having a longitudinal direction (L) and a transverse direction (T). The sheet has an inner surface 2 and an outer surface 3, the inner surface comprising an edge zone 4 comprising an inner edge portion 5, 8 and an outer edge portion 6, 7. The packaging unit 1 also comprises transverse edge portions 4' and 4". The sheet has a longitudinal center line (L1) and a folding axis 9, wherein the folding axis divides the sheet into a first region 10 and a second region 11. As shown in Fig. 1, the outer edge portion 6 of the edge zone 4 of the first region 10 is provided with resealable adhesive 12, while the inner edge portion 5 of the edge zone 4 of the first region 10 is adhesive-free. Further, the inner edge portion 8 of the edge zone 4 of the second region 11 is provided with resealable adhesive 12, while the outer edge portion 7 of the edge zone 4 of the second region 11 is adhesive-free. Also, the transverse edge portion 4' of the first region 10 is provided with resealable adhesive 12, while the transverse edge portion 4" of the second region 11 is adhesive-free. Thus, the resealable adhesive pattern in the first region 10 is complementary to the resealable adhesive pattern of the second region 11. This in turn means that, when the sheet is folded about the folding axis 9 as shown in Fig. 2, the edge portions 6 carrying resealable adhesive in the first region 10 are brought in contact with the adhesive-free edge portions 7 in the second region 11, the edge portions 8 carrying resealable adhesive 12 in the second region 11 are brought in contact with the adhesive-free edge portions 5 in the first region 10, and the transverse edge portion 4' carrying resealable adhesive 12 in the first region 10 is brought in contact with the adhesive-free transverse edge portion 4" in the second region 11. As can be seen from Fig. 2, the width of the sealing area corresponds to the sum of the widths of the resealable adhesive portions 6, 8. Such a sealing ensures a tight package for both a new and a disposed article.

Accordingly, the resealable adhesive (-s) is provided on edge portions such that when the sheet is folded about the folding axis 9, the resealable adhesive aids in the closure of the packaging unit 1 enclosing a hygiene article in a tight and hygienic way. Fig. 2 illustrates the folded sheet along the at least one folding axis with the first and second regions 10, 12 in an overlapping configuration, thereby providing a packaging unit 1 of overlapping sheet layers.

A set forth in Figs. 1 and 2, the sheet is further provided with two sync marks 13 for indication of a positioning of the sheet of material during manufacturing of the packaging unit 1. Each sync mark 13 is provided on the outer surface 3 of the sheet at a corner portion of the first region 10. By providing the sync mark 13 on the outer surface 2 of the sheet, an improved adhesive connection to the inner surface 2 is made possible. This is because adhesive bonds less well to a printed surface. With no sync marks on the inner surface 2, the inner surface 2 can be prepared for pre-treatments, such as corona treatments, for providing an improved adhesive connection to the inner surface 4, as described herein.

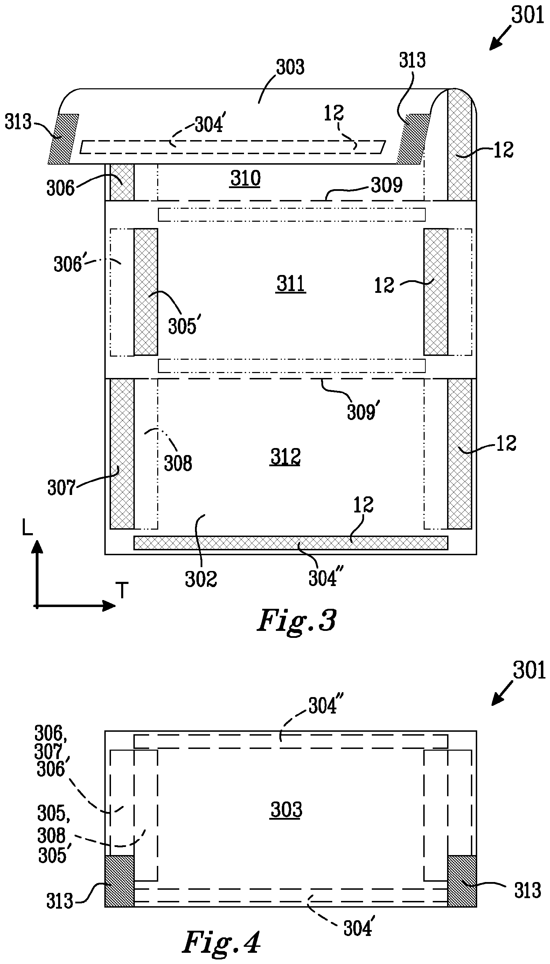

The most common packaging unit for individual packaging of absorbent articles is a rectangular sheet comprising two folding axes, longitudinal edges and transverse edges. Such an embodiment is illustrated in Fig. 3. The packaging unit 301 is a rectangular sheet comprising two folding axes 309, 309' dividing the packaging unit into a first region 310, a second region 311 and a third region 312. Each of the regions comprises an inner edge portion 305, 305', 308 and an outer edge portion 306, 306', 307. As shown in Fig. 3, the outer edge portions 306, 307 of the first and third regions respectively are provided with resealable adhesive 12, while the inner edge portions 305, 308 of the first and third regions respectively are adhesive-free. The resealable adhesive pattern of the second region 311 is complementary to the resealable adhesive pattern of the first and third regions 310, 312, thus forming a chessboard pattern. In other words, the outer edge portion 306' of the second region 311 is adhesive-free, and the inner edge portion 305' of the second region 311 is provided with resealable adhesive. This in turn means that, when the sheet is e-folded about the folding axes 309, 309' as shown in Fig. 4, the outer edge portions 306 of the first region 310 or the outer edge portions 307 of the third region 312 carrying resealable adhesive 12 are brought in contact with the adhesive-free outer edge portions 306' in the second region 311, depending on which of the first and the third regions 310, 312 is brought in contact with the second region 311. Consequently, the inner edge portions 305' carrying resealable adhesive 12 in the second region 311 are brought into contact with the adhesive-free inner edge portions 305 of the first region 310 or the adhesive-free inner edge portions 308 of the third region 312.

As can be seen from Fig. 4, the width of the sealing area corresponds to the sum of the widths of the resealable adhesive portions 306, 305'. It should be noted that the order in which the packaging unit is folded is irrelevant. For example, the packaging unit may be folded around the second folding axis 309', bringing the third region 312 in contact with the second region 311, sealing the outer edge portions 307, 306' and the inner edge portions 308, 305'. The packaging unit is subsequently folded around the first folding axis 309', bringing the first region 310 in contact with the outer surface of the third region 312, thus sealing the packaging unit (Fig. 4). The folding order may also be reversed. This is a great advantage, since when the packaging unit of the present invention is used for disposal; the user does not have to fold the packaging unit in any particular order to be able to obtain a tightly sealed package. Thus, the packaging unit will provide a tight and hygienic package regardless of the folding order.

In order to obtain a tight package, the transverse edge portions 304' and 304" of the packaging unit 301 are provided with resealable adhesive 12. When both transverse edges 304' and 304" are provided with resealable adhesive, the folding order is irrelevant, as described above. It is also conceivable to provide only one of the transverse edges with resealable adhesive. In this case, the folding should be initiated around the folding axis being positioned closest to the adhesive-free transverse edge, such that the region comprising the adhesive-covered transverse edge portion forms a lid and the resealable adhesive positioned at the transverse edge portion seals the packaging unit.

The sheet as set forth in Figs. 3 and 4 is also provided with two sync marks 313 for indication of a positioning of the sheet of material during manufacturing of the packaging unit. Each sync mark 313 is provided on the outer surface 303 of the sheet at a corner portion of the first region 310. As for the single-axis sheet, the provision of the sync mark 313, such as a printed sync mark, on the outer surface 303 means that an improved adhesive connection to the inner surface 302 is made possible as further described herein.

The use of marks for indicating, for example, cutting positions of a product is known from, for example, WO 03030796 A1.

It should be noted that when the packaging unit according to the present invention is used for disposal, the user may choose to roll up the packaging unit 1, 301 with the soiled article positioned on it rather than folding it.

The packaging unit is intended to include a hygiene article. The hygiene article may be arranged on the inner surface, in the center zone thereof, wherein the hygiene article may be attached to the release agent via an adhesive or to a release liner(not shown), as now will be further elaborated on in the described example of a method of forming the packaging unit.

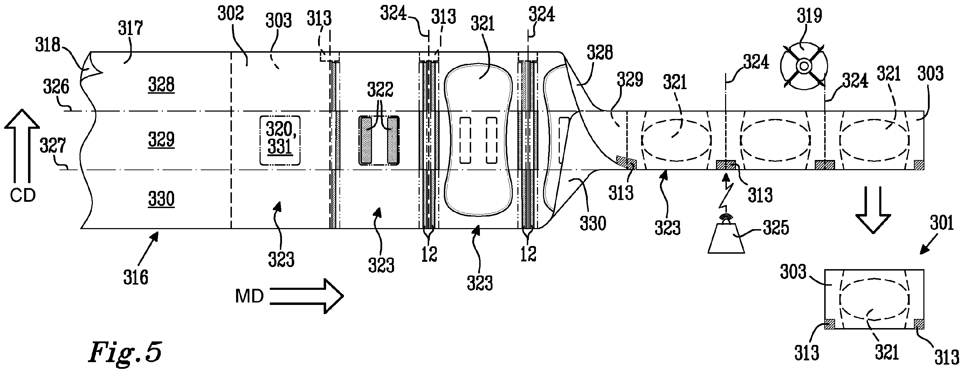

The packaging units may be formed from a blank. Fig. 5 schematically illustrates an arrangement of using a blank 316 for forming a packaging unit 301 with two folding axes 309, 309' in accordance with the packaging unit 301 shown in Figs. 3 and 4. The blank 316 is an elongated sheet of material having first and second surfaces 317, 318, wherein the first surface comprises the inner surface 302 of each packaging unit 301 and the second surface 318 comprises the outer surface 303 of each packaging unit 301. The inner surface is provided with resealable adhesive 12 for closure of the packaging unit 301 formed from the blank 316, and the outer surface 303 is provided with one or more sync marks 313. As mentioned above, such a sync mark 313 may be used for guiding a cutting tool 319 to cut the blank 316 into individual sheets of material and forming the packaging units 301.

A release agent 320 or a release liner may be applied on the first surface 317 and inner surface 302, and a hygiene article 321 may be arranged on the release agent 320 via an adhesive 322 or on the release liner via fastening means.

At least a portion of the first surface 317 and the inner surface 302 may be pre-treated with a surface treatment for increasing the surface energy before any adhesive has been applied on the first surface so as to improve adhesive connection to the pre-treated portion of the first surface 317 and the inner surface 302. The surface treatment may be a plasma treatment, such as a corona treatment as is described in more detail herein above.

The method used in the arrangement as illustrated in Fig. 5 then comprises the steps of:
- providing a blank 316 of an elongated sheet of material comprising at least two packaging unit sections 323, the sheet having first and second surfaces 317, 318, the first surface 317 comprising an inner surface 302 of each packaging unit 301 and the second surface 318 comprising an outer surface 303 of each packaging unit 301,
- providing the outer surface 303 with one or more sync marks 313, e.g. one or more printed marks;
- applying a resealable adhesive 12 on an edge zone portion of the inner surface 302 of each packaging unit section 323;
- detecting the sync mark 313 and its position on the blank 316, and
- cutting the elongated sheet along a cutting line 324 into two or more packaging units 301.

The position of cutting and thus the cutting line may be determined based on the detected position of the sync mark (-s) 313.

In Fig. 5, each sync mark 313 is provided on the outer surface 303 of the sheet at a corner portion of the first region 310. Each sync mark 313 is applied on the blank as a combined sync mark overlapping the cutting line 324, and thus the combined sync mark forms two sync marks 313 after the blank 326 has been cutted, wherein a sync mark 313 is formed on each packaging unit.

Each portion with resealable adhesive 12 may be arranged in a zone close to and along the cutting line 324 of a packaging unit section 323, wherein the resealable adhesive 12 in some portions may be applied as one adhesive string (not shown) along and overlapping the cutting line 323 so that the applied string forms a portion with resealable adhesive 12 on each one of two packaging units 301 after cutting has taken place (not shown). The resealable adhesive 12 may also be separately applied to each packaging unit section 323 at a distance from the cutting line 324.

One or more sync mark detectors may be arranged to detect sync marks on the outer surfaces 303 during the manufacturing, wherein detection of the sync marks is used for indicating a position of a blank 316 sheet of material so as to determine, for example, a cutting position of the blank sheet of material for providing single packaging unit such as a wrap. Fig. 5 illustrates one detector 325.

As mentioned herein above, at least a portion of the first surface 317 may be pre-treated with a surface treatment for increasing the surface energy The surface treatment may be a plasma treatment. The treatment may be a corona treatment.

The elongated sheet may comprise at least one folding axis 326, 327 extending in the machine direction (MD). In Fig. 5, the elongated sheet has two folding axes 326, 327 dividing the elongated sheet into a first blank region 328, a second blank region 329 and a third blank region 330. The method illustrated in Fig. 5 further comprises folding the elongated sheet along the folding axes 326, 327 with the blank regions in an overlapping configuration, thereby providing a folded elongated sheet of overlapping sheet layers, wherein the step of cutting the elongated sheet occurs after the folding step. The transversely extending folding axes 326, 327 extend along the machine direction (MD) and the cutting occurs along the cross machine direction (CD). The folding is provided by any folding tool as is well known in the art (not shown).

The cutting may also occur prior to the folding step (not shown). Nonetheless, the transversely extending folding axis may still extend along the machine direction (MD) and the cutting may occur along the cross machine direction (CD).

Prior to the folding step, the method as illustrated in Fig. 5 further comprises:
- applying a release agent 320 on the first surface 317 on a portion of each center zone 331 of a packaging unit section 323 (and corresponding packaging unit 301),
- applying an adhesive 322 on the portion with release agent 320, and
- applying a hygiene article 321 to the adhesive 322 applied on the release agent 320.

The sync mark (-s) 313 may also aid in positioning of the applied agents and articles.

As an alternative to use a release agent 320 and on the first surface 317 as illustrated in Fig. 5, the method may, prior to the folding step, further comprise:
- attaching a release liner on the first surface 317 on a portion of each center zone 331 of a packaging unit section 323 and
- connecting a hygiene article 321 to the release liner by the use of fastening means.

As the skilled person will appreciate, it is intended that the detailed description be regarded as illustrative and that many embodiments and alternatives are possible within the scope of the present invention as defined by the appended claims. For example, the packaging unit may adopt other shapes than the rectangular ones shown in the drawings. Furthermore, the invention has been exemplified with packaging units comprising resealable adhesive for closure of the packaging unit, wherein a chessboard pattern of resealable adhesive and non-adhesive portions is formed along at least longitudinal edge zones (i.e. along the longitudinal direction (L)) for complementary attachments between resealable adhesive and non-adhesive portions in a folded packaging unit. Other ways of applying one or more resealable adhesive portions for closure of the packaging unit may also be considered.

## Claims

1. A packaging unit for hygiene articles (1, 301), the unit being formed from a sheet of material having a longitudinal direction (L) and a transverse direction (T), said sheet having an inner surface (2, 302) and an outer surface (3, 303), said inner surface (2, 302) comprising an edge zone that comprises an edge portion (4', 4", 6, 8, 304, 304', 306, 305', 307) being provided with a resealable adhesive (12) for closure of the packaging unit (1, 301), said sheet having at least one transversely extending folding axis (9, 309, 309'), said folding axis (9, 309, 309') dividing said sheet into a first region (10, 310) and a second region (11, 311), said sheet is provided with at least one sync mark (13, 313), such as a printed mark, for indication of a positioning of the sheet of material during manufacturing of the packaging unit (1, 301), said sync mark (13, 313) being provided on the outer surface (3, 303), **characterized in that** said sheet of material is of substantially rectangular shape and comprises longitudinal edges (14, 15; 314, 315) and transverse edges (16, 17; 314, 315), the sync mark (13, 313) being provided at a longitudinal edge portion and/or at a transversal edge portion.

2. Packaging unit according to claim 1, wherein at least a portion of the inner surface (2, 302) is pre-treated with a surface treatment, such as a plasma treatment, preferably a corona treatment, for changing the surface energy.

3. Packaging unit according to claim 2, wherein a portion of said resealable adhesive (12) is in contact with a portion of the surface (2, 302) that has been pre-treated with surface treatment.

4. The packaging unit to any one of the preceding claims, wherein said sheet is folded along said at least one folding axis (9, 309, 309') with said first and second regions (10, 11; 310, 311) in an overlapping configuration, thereby providing a packaging unit (1, 301) of overlapping sheet layers.

5. The packaging unit according to any one of the preceding claims, wherein said sheet has two folding axes (309, 309'), dividing said sheet into said first region (310), said second region (311) and a third region (312), said sheet being folded along said folding axes (309, 309') with said first, second and third regions (310, 311,312) in an overlapping configuration of three layers.

6. The packaging unit according to claim 5, wherein the first region (310) forms an outer layer of the folded packaging unit and the third region (312) an inner layer, wherein the sync mark (313) is provided on the first region, the second region or the third region.

7. The packaging unit according to any one of the preceding claims, wherein the sheet is a single-ply material, such as a single-ply material of polyolefin, preferably a polyolefin comprising or being of polyethylene and/or polypropylene, more preferably a material of polyethylene and/or polypropylene that has a basis weight of 14 to 30 g/m2.

8. The packaging unit according to any one of the preceding claims, wherein a chessboard pattern of resealable adhesive (12) and non-adhesive portions is formed along the edge zone (4) for complementary attachment between resealable adhesive (12) and non-adhesive portions in a folded packaging unit.

9. The packaging unit according to any one of the preceding claims, wherein said sheet is opaque and/or comprises print and/or tactile indications other than the sync mark (13, 313), preferably provided on the outer surface.

10. The packaging unit according to any one of the preceding claims, wherein said resealable adhesive (12) is a pressure-sensitive hot melt adhesive.

11. The packaging unit according to any one of the preceding claims, wherein said packaging unit (1, 301) is reclosable.

12. The packaging unit according to any one of the preceding claims, wherein the inner surface (2, 302) comprises a center zone (331) that comprises portion provided with a release agent (320) or a release liner.

13. A blank (316) for manufacturing at least two packaging units (1, 301), each packaging unit (1, 301) being defined as claimed in any one of the preceding claims, wherein the blank (316) is an elongated sheet of material having first and second surfaces (317, 318), the first surface (317) comprising the inner surface (2, 302) of each packaging unit (1, 301) and the second surface (318) comprising the outer surface (3, 303) of each packaging unit(1, 301), wherein said inner surface (2, 302) comprises a portion with resealable adhesive (12) for closure of the packaging unit (1, 301) formed from the blank (316) and wherein said outer surface (3, 303) comprises one or more sync marks (13, 313).

14. The blank according to claim 13, wherein at least a portion of the inner surface (2, 302) is pre-treated with a surface treatment for increasing the surface energy.

15. A method of forming a packaging unit for hygiene articles in accordance with any one of the preceding claims 1 to 12, the unit (1, 301) being formed from a blank (316) of an elongated sheet of material, the method comprising the steps of:
- providing said elongated sheet of material comprising at least two packaging unit sections (323), said sheet having first and second surfaces (317, 318), the first surface (317) comprising an inner surface (2, 302) of each packaging unit (1, 301) and the second surface (318) comprising an outer surface (3, 303) of each packaging unit (1, 301),
- providing said outer surface (2, 302) with one or more sync marks (13, 313),
- applying a resealable adhesive (12) on an edge zone portion (4', 4", 6, 8, 304, 304', 306, 305', 307) of the inner surface (2, 302) of each packaging unit section (1, 301), optionally pre-treating at least a portion of the first surface (317) with a surface treatment, such as a plasma treatment, preferably a corona treatment, for increasing the surface energy before said resealable adhesive (12) is applied thereon;
- detecting the sync mark (13, 313) and its position on the blank (316), and
- cutting said elongated sheet into two or more single sheets or packaging units (1, 301).

16. The method according to claim 15, wherein the elongated sheet comprises at least one folding axis (326, 327) extending in the machine direction (MD), said folding axis (326, 327) dividing said elongated sheet into a first blank region (328) and a second blank region (329), wherein the method further comprises folding said elongated sheet along said at least one folding axis (326, 327) with said first and second blank regions (328, 329) in an overlapping configuration, thereby providing a folded elongated sheet of overlapping sheet layers, wherein said step of cutting the elongated sheet occurs after the folding step.

17. The method according to claim 15, wherein each single sheet formed after said step of cutting comprises at least one transversely extending folding axis (326, 327), said folding axis (326, 327) dividing each single sheet into a first region (328) and a second region (329), wherein the method following the method step of cutting the elongated sheet further comprises folding said single sheet along said at least one folding axis (326, 327) with said first and second regions (328, 329) in an overlapping configuration, thereby providing a packaging unit of overlapping sheet layers.

18. The method according to any one of claims 15-17, wherein the method, prior to the folding step, further comprises
- applying a release agent (320) on said first surface on a portion of each center zone (331) of a packaging unit section,
- applying an adhesive (322) on the portion with release agent (320), and
- applying a hygiene article (321) to said adhesive (322) applied on the release agent (320).

19. The method according to any one of claims15 to 18, wherein the method, prior to the folding step, further comprises:
- attaching a release liner on the first surface on a portion of each center zone (331) of a packaging unit section and
- connecting a hygiene article (321) to the release liner by the use of fastening means.

## Patentansprüche

1. Verpackungseinheit für Hygieneartikel (1, 301), wobei die Einheit aus einem Materialbogen ausgebildet ist, der eine Längsrichtung (L) und eine Querrichtung (T) aufweist, der Bogen eine innere Fläche (2, 302) und eine äußere Fläche (3, 303) aufweist, die innere Fläche (2, 302) eine Kantenzone aufweist, die einen Kantenabschnitt (4', 4'', 6, 8, 304, 304', 306, 305', 307) aufweist, der mit einem wiederversiegelbaren Haftmittel (12) zum Schließen der Verpackungseinheit (1, 301) versehen ist, der Bogen mindestens eine sich in Querrichtung erstreckende Faltachse (9, 309, 309') aufweist, die Faltachse (9, 309, 309') den Bogen in einen ersten Bereich (10, 310) und einen zweiten Bereich (11, 311) unterteilt, der Bogen mit mindestens einer Synchronisationsmarkierung (13, 313), wie zum Beispiel eine gedruckte Markierung, zum Kennzeichnen einer Positionierung des Materialbogens während eines Herstellens der Verpackungseinheit (1, 301) versehen ist und die Synchronisationsmarkierung (13, 313) an der äußeren Fläche (3, 303) vorgesehen ist, **dadurch gekennzeichnet, dass** der Materialbogen eine im Wesentlichen rechtwinklige Form und Längskanten (14, 15; 314, 315) und Querkanten (16, 17; 314, 315) aufweist, wobei die Synchronisationsmarkierung (13, 313) bei einem Längskantenabschnitt und/oder bei einem Querkantenabschnitt vorgesehen sind.

2. Verpackungseinheit nach Anspruch 1, bei der zumindest ein Abschnitt der inneren Fläche (2, 302) zum Ändern der Flächenenergie mit einer Flächenbehandlung, wie zum Beispiel eine Plasmabehandlung, vorzugsweise eine Koronabehandlung, vorbehandelt ist.

3. Verpackungseinheit nach Anspruch 2, bei der ein Abschnitt des wiederversiegelbaren Haftmittels (12) mit einem Abschnitt der Fläche (2, 302) in Kontakt ist, der mit einer Flächenbehandlung vorbehandelt worden ist.

4. Verpackungseinheit nach einem der vorstehenden Ansprüche, bei welcher der Bogen entlang der mindestens einen Faltachse (9, 309, 309') mit dem ersten und zweiten Bereich (10, 11; 310, 311) in eine überlappende Anordnung gefaltet ist, um dadurch eine Verpackungseinheit (1, 301) überlappender Bogenlagen bereitzustellen.

5. Verpackungseinheit nach einem der vorstehenden Ansprüche, bei welcher der Bogen zwei Faltachsen (309, 309') aufweist, welche den Bogen in den ersten Bereich (310), den zweiten Bereich (311) und einen dritten Bereich (312) unterteilen, wobei der Bogen entlang der Faltachse (309, 309') mit dem ersten, zweiten und dritten Bereich (310, 311, 312) in einer überlappenden Anordnung dreier Lagen gefaltet ist.

6. Verpackungseinheit nach Anspruch 5, bei welcher der erste Bereich (310) eine äußere Lage der gefalteten Verpackungseinheit und der dritte Bereich (312) eine innere Lage ausbildet, wobei die Synchronisationsmarkierung (313) an dem ersten Bereich, dem zweiten Bereich oder dem dritten Bereich vorgesehen ist.

7. Verpackungseinheit nach einem der vorstehenden Ansprüche, bei welcher der Bogen ein einlagiges Material ist, wie zum Beispiel ein einlagiges Material aus Polyolefin, vorzugsweise ein Polyolefin mit oder aus Polyethylen und/oder Polypropylen, noch bevorzugter ein Material aus Polyethylen und/oder Polypropylen, das ein Flächengewicht von 14-30 g/m² aufweist.

8. Verpackungseinheit nach einem der vorstehenden Ansprüche, bei der ein Schachmuster eines wiederversiegelbaren Haftmittels (12) und nicht haftende Abschnitte entlang der Kantenzone (4) für ein komplementäres Anbringen zwischen wiederversiegelbarem Haftmittel (12) und nicht haftenden Abschnitten in einer gefalteten Verpackungseinheit ausgebildet ist.

9. Verpackungseinheit nach einem der vorstehenden Ansprüche, bei welcher der Bogen lichtundurchlässig ist und/oder einen Druck und/oder andere taktile Anzeigen aufweist als die Synchronisationsmarkierung (13, 313), die vorzugsweise an der äußeren Fläche vorgesehen sind.

10. Verpackungseinheit nach einem der vorstehenden Ansprüche, bei der das wiederversiegelbare Haftmittel (12) ein druckempfindliches Heißschmelzhaftmittel ist.

11. Verpackungseinheit nach einem der vorstehenden Ansprüche, bei der die Verpackungseinheit (1, 301) wiederverschließbar ist.

12. Verpackungseinheit nach einem der vorstehenden Ansprüche, bei der die innere Fläche (2, 302) eine mittige Zone (331) aufweist, die einen Abschnitt aufweist, der mit einem Trennmittel (320) oder einer Trennschicht versehen ist.

13. Rohling (316) zum Herstellen mindestens zweier Verpackungseinheiten (1, 301), wobei jede Verpackungseinheit (1, 301) wie in einem der vorstehenden Ansprüche beansprucht definiert ist, der Rohling (316) ein länglicher Materialbogen mit einer ersten und einer zweiten Fläche (317, 318) ist, die erste Fläche (317) die innere Fläche (2, 302) jeder Verpackungseinheit (1, 301) und die zweite Fläche (318) die äußere Fläche (3, 303) jeder Verpackungseinheit (1, 301) aufweist, die innere Fläche (2, 302) einen Abschnitt mit wiederversiegelbarem Haftmittel (12) zum Schließen der Verpackungseinheit (1, 301) aufweist, die aus dem Rohling (316) ausgebildet ist und die äußere Fläche (3, 303) eine oder mehrere Synchronisationsmarkierungen (3, 313) aufweist.

14. Rohling nach Anspruch 13, bei dem zumindest ein Abschnitt der inneren Fläche (2, 302) zum Erhöhen der Flächenenergie mit einer Flächenbehandlung vorbehandelt ist.

15. Verfahren zum Ausbilden einer Verpackungseinheit für Hygieneartikel nach einem der vorstehenden Ansprüche 1 bis 12, wobei die Einheit (1, 301) aus einem Rohling (316) aus einem länglichen Materialbogen ausgebildet ist, und das Verfahren die Schritte umfasst:
- Bereitstellen des länglichen Materialbogens, der mindestens zwei Verpackungseinheitabschnitte (323) aufweist, wobei der Bogen eine erste und eine zweite Fläche (317, 318) aufweist, die erste Fläche (317) eine innere Fläche (2, 302) jeder Verpackungseinheit (1, 301) aufweist und die zweite Fläche (318) eine äußere Fläche (3, 303) jeder Verpackungseinheit (1, 301) aufweist,
- Bereitstellen der äußeren Fläche (2, 302) mit einer oder mehreren Synchronisationsmarkierungen (13, 313),
- Aufbringen eines wiederversiegelbaren Haftmittels (12) auf einen Kantenzonenabschnitt (4', 4'', 6, 8, 304, 304', 306, 305', 307) der inneren Fläche (2, 302) von jedem Verpackungseinheitabschnitt (1, 301), wobei optional mindestens ein Abschnitt der ersten Fläche (317) mit einer Flächenbehandlung, wie zum Beispiel einer Plasmabehandlung, vorzugsweise einer Koronabehandlung, zum Erhöhen der Flächenenergie vorbehandelt wird, bevor das wiederversiegelbare Haftmittel (12) darauf aufgebracht wird;
- Erfassen der Synchronisationsmarkierung (13, 313) und ihrer Position an dem Rohling (316), und
- Schneiden des länglichen Bogens in zwei oder mehrere einzelne Bögen oder Verpackungseinheiten (1, 301).

16. Verfahren nach Anspruch 15, bei dem der längliche Bogen mindestens eine Faltachse (326, 327) aufweist, die sich in der Maschinenrichtung (MD) erstreckt, wobei die Faltachse (326, 327) den länglichen Bogen in einen ersten Rohlingsbereich (328) und einen zweiten Rohlingsbereich (329) unterteilt, wobei das Verfahren ferner ein Falten des länglichen Bogens entlang der mindestens einen Faltachse (326, 327) mit dem ersten und zweiten Rohlingsbereich (328, 329) in einer überlappenden Anordnung umfasst, um dadurch einen gefalteten länglichen Bogen sich überlappender Bogenlagen bereitzustellen, wobei der Schritt eines Schneidens des länglichen Bogens nach dem Faltschritt auftritt.

17. Verfahren nach Anspruch 15, bei dem jeder nach dem Schneideschritt ausgebildete einzelne Bogen mindestens eine sich quer erstreckende Faltachse (326, 327) aufweist, wobei die Faltachse (326, 327) jeden einzelnen Bogen in einen ersten Bereich (328) und einen zweiten Bereich (329) unterteilt, wobei das Verfahren auf den Verfahrensschritt des Schneidens des länglichen Bogens folgend ferner ein Falten des einzelnen Bogens entlang der mindestens einen Faltachse (326, 327) mit dem ersten und zweiten Bereich (328, 329) in einer überlappenden Anordnung umfasst, um dadurch eine Verpackungseinheit überlappender Bogenlagen bereitzustellen.

18. Verfahren nach einem der Ansprüche 15-17, wobei das Verfahren vor dem Faltschritt ferner umfasst:
- Aufbringen eines Trennmittels (320) auf die erste Fläche auf einen Abschnitt jeder mittigen Zone (331) eines Verpackungseinheitabschnitts,
- Aufbringen eines Haftmittels (322) auf den Abschnitt mit dem Trennmittel (320), und
- Aufbringen eines Hygieneartikels (321) auf das Haftmittel (322), das auf das Trennmittel (320) aufgebracht worden ist.

19. Verfahren nach einem der Ansprüche 15-18, wobei das Verfahren vor dem Faltschritt ferner umfasst:
- Anbringen einer Trennschicht auf die erste Fläche auf einen Abschnitt von jeder mittigen Zone (331) eines Verpackungseinheitabschnitts und
- Verbinden eines Hygieneartikels (321) mit der Trennschicht durch eine Verwendung eines Befestigungsmittels.

## Revendications

1. Unité de conditionnement pour articles d'hygiène (1, 301), l'unité étant formée d'une feuille de matériau ayant une direction longitudinale (L) et une direction transversale (T), ladite feuille ayant une surface interne (2, 302) et une surface externe (3, 303), ladite surface interne (2, 302) comprenant une zone de bord qui comprend une portion de bord (4', 4", 6, 8, 304, 304', 306, 305', 307) étant dotée d'un adhésif décollable (12) pour la fermeture de l'unité de conditionnement (1, 301), ladite feuille ayant au moins un axe de pliage s'étendant de manière transversale (9, 309, 309'), ledit axe de pliage (9, 309, 309') divisant ladite feuille en une première région (10, 310) et une deuxième région (11, 311), ladite feuille est dotée d'au moins une marque de synchronisation (13, 313), telle qu'une marque imprimée, pour l'indication d'un positionnement de la feuille de matériau durant la fabrication de l'unité de conditionnement (1, 301), ladite marque de synchronisation (13, 313) étant fournie sur la surface externe (3, 303), **caractérisée en ce que** ladite feuille de matériau est de forme sensiblement rectangulaire et comprend des bords longitudinaux (14, 15 ; 314, 315) et des bords transversaux (16, 17 ; 314, 315), la marque de synchronisation (13, 313) étant fournie au niveau d'une portion de bord longitudinal et/ou au niveau d'une portion de bord transversal.

2. Unité de conditionnement selon la revendication 1, dans laquelle au moins une portion de la surface interne (2, 302) est prétraitée par un traitement de surface, tel qu'un traitement par plasma, de préférence un traitement corona, pour changer l'énergie de surface.

3. Unité de conditionnement selon la revendication 2, dans laquelle une portion dudit adhésif décollable (12) est en contact avec une portion de la surface (2, 302) qui a été prétraitée par traitement de surface.

4. Unité de conditionnement selon l'une quelconque des revendications précédentes, dans laquelle ladite feuille est pliée le long dudit au moins un axe de pliage (9, 309, 309') avec lesdites première et deuxième régions (10, 11 ; 310, 311) dans une configuration en chevauchement, fournissant ainsi une unité de conditionnement (1, 301) de couches de feuilles en chevauchement.

5. Unité de conditionnement selon l'une quelconque des revendications précédentes, dans laquelle ladite feuille a deux axes de pliage (309, 309'), divisant ladite feuille en ladite première région (310), ladite deuxième région (311) et une troisième région (312), ladite feuille étant pliée le long desdits axes de pliage (309, 309') avec lesdites première, deuxième et troisième régions (310, 311, 312) dans une configuration en chevauchement de trois couches.

6. Unité de conditionnement selon la revendication 5, dans laquelle la première région (310) forme une couche externe de l'unité de conditionnement pliée et la troisième région (312) une couche interne, dans laquelle la marque de synchronisation (313) est fournie sur la première région, la deuxième région ou la troisième région.

7. Unité de conditionnement selon l'une quelconque des revendications précédentes, dans laquelle la feuille est un matériau à pli unique, tel qu'un matériau à pli unique de polyoléfine, de préférence une polyoléfine comprenant ou étant constituée de polyéthylène et/ou de polypropylène, de manière davantage préférée un matériau de polyéthylène et/ou de polypropylène qui a un poids de base de 14 à 30 g/m2.

8. Unité de conditionnement selon l'une quelconque des revendications précédentes, dans laquelle un motif en échiquier d'adhésif décollable (12) et de portions non adhésives est formé le long de la zone de bord (4) pour une fixation complémentaire entre l'adhésif décollable (12) et les portions non adhésives dans une unité de conditionnement pliée.

9. Unité de conditionnement selon l'une quelconque des revendications précédentes, dans laquelle ladite feuille est opaque et/ou comprend une impression et/ou des indications tactiles autres que la marque de synchronisation (13, 313), de préférence fournies sur la surface externe.

10. Unité de conditionnement selon l'une quelconque des revendications précédentes, dans laquelle ledit adhésif décollable (12) est un adhésif thermofusible sensible à la pression.

11. Unité de conditionnement selon l'une quelconque des revendications précédentes, dans laquelle ladite unité de conditionnement (1, 301) est refermable.

12. Unité de conditionnement selon l'une quelconque des revendications précédentes, dans laquelle la surface interne (2, 302) comprend une zone centrale (331) qui comprend une portion dotée d'un agent antiadhérent (320) ou d'une bande antiadhérente.

13. Ébauche (316) pour fabriquer au moins deux unités de conditionnement (1, 301), chaque unité de conditionnement (1, 301) étant définie selon l'une quelconque des revendications précédentes, dans laquelle l'ébauche (316) est une feuille allongée de matériau ayant des première et seconde surfaces (317, 318), la première surface (317) comprenant la surface interne (2, 302) de chaque unité de conditionnement (1, 301) et la seconde surface (318) comprenant la surface externe (3, 303) de chaque unité de conditionnement (1, 301), dans laquelle ladite surface interne (2, 302) comprend une portion présentant un adhésif décollable (12) pour la fermeture de l'unité de conditionnement (1, 301) formée à partir de l'ébauche (316) et dans laquelle ladite surface externe (3, 303) comprend une ou plusieurs marques de synchronisation (13, 313).

14. Ébauche selon la revendication 13, dans laquelle au moins une portion de la surface interne (2, 302) est prétraitée par un traitement de surface pour augmenter l'énergie de surface.

15. Procédé de formation d'une unité de conditionnement pour articles d'hygiène selon l'une quelconque des revendications précédentes 1 à 12, l'unité (1, 301) étant formée à partir d'une ébauche (316) d'une feuille allongée de matériau, le procédé comprenant les étapes de :
- fourniture de ladite feuille allongée de matériau comprenant au moins deux sections d'unité de conditionnement (323), ladite feuille ayant des première et seconde surfaces (317, 318), la première surface (317) comprenant une surface interne (2, 302) de chaque unité de conditionnement (1, 301) et la seconde surface (318) comprenant une surface externe (3, 303) de chaque unité de conditionnement (1, 301),
- fourniture sur ladite surface externe (2, 302) d'une ou plusieurs marques de synchronisation (13, 313),
- application d'un adhésif décollable (12) sur une portion de zone de bord (4', 4", 6, 8, 304, 304', 306, 305', 307) de la surface interne (2, 302) de chaque section d'unité de conditionnement (1, 301), facultativement en prétraitant au moins une portion de la première surface (317) par un traitement de surface, tel qu'un traitement par plasma, de préférence un traitement corona, pour augmenter l'énergie de surface avant que ledit adhésif décollable (12) ne soit appliqué sur celle-ci ;
- détection de la marque de synchronisation (13, 313) et de sa position sur l'ébauche (316), et
- découpage de ladite feuille allongée en deux feuilles ou unités de conditionnement (1, 301) individuelles ou plus.

16. Procédé selon la revendication 15, dans lequel la feuille allongée comprend au moins un axe de pliage (326, 327) s'étendant dans le sens machine (MD), ledit axe de pliage (326, 327) divisant ladite feuille allongée en une première région d'ébauche (328) et une seconde région d'ébauche (329), dans lequel le procédé comprend en outre le pliage de ladite feuille allongée le long dudit au moins un axe de pliage (326, 327) avec lesdites première et seconde régions d'ébauche (328, 329) dans une configuration en chevauchement, fournissant ainsi une feuille allongée pliée de couches de feuilles en chevauchement, dans lequel ladite étape de découpage de la feuille allongée a lieu après l'étape de pliage.

17. Procédé selon la revendication 15, dans lequel chaque feuille individuelle formée après ladite étape de découpage comprend au moins un axe de pliage s'étendant de manière transversale (326, 327), ledit axe de pliage (326, 327) divisant chaque feuille individuelle en une première région (328) et une deuxième région (329), dans lequel le procédé suivant l'étape de procédé de découpage de la feuille allongée comprend en outre le pliage de ladite feuille individuelle le long dudit au moins un axe de pliage (326, 327) avec lesdites première et deuxième régions (328, 329) dans une configuration en chevauchement, fournissant ainsi une unité de conditionnement de couches de feuilles en chevauchement.

18. Procédé selon l'une quelconque des revendications 15 à 17, dans lequel le procédé, avant l'étape de pliage, comprend en outre
- l'application d'un agent antiadhérent (320) sur ladite première surface sur une portion de chaque zone centrale (331) d'une section d'unité de conditionnement,
- l'application d'un adhésif (322) sur la portion présentant un agent antiadhérent (320), et
- l'application d'un article d'hygiène (321) sur ledit adhésif (322) appliqué sur l'agent antiadhérent (320).

19. Procédé selon l'une quelconque des revendications 15 à 18, dans lequel le procédé, avant l'étape de pliage, comprend en outre :
- la fixation d'une bande antiadhérente sur la première surface sur une portion de chaque zone centrale (331) d'une section d'unité de conditionnement et
- la liaison d'un article d'hygiène (321) à la bande antiadhérente par l'utilisation d'un moyen d'attache.
